# EUROPEAN PATENT APPLICATION

(11) **EP 3 260 551 A1**
(43) Date of publication of application: **27.12.2017**
(21) Application number: 16197377.1
(22) Date of filing: 19.06.2008
(51) Int. Cl.: C12Q 1/04, A61K 31/35

(54) **USE OF (1R,2S,5R) 1,6-DIAZABICYCLO [3.2.1] OCTANE-2-CARBOXAMIDE, 7-OXO-6-(SULFOOXY)-, MONOSODIUM SALT AS A DIAGNOSTIC REAGENT FOR DETECTING SERINE BETA-LACTAMASES**

(62) Divisional of application: 08290582.9
(71) Applicant: ASTRA ZENECA HOLDING FRANCE, 92500 Rueil Malmaison (FR)
(72) Inventor: SHLAES, David, 93230 Romainville (FR); LEVASSEUR, Premavathy, 95880 Enghien les Bains (FR)
(74) Representative: Hirsch & Associés

(57) **Abstract**

Use of (1R,2S,5R) 1,6-diazabicyclo[3.2.1]octane-2-carboxamide, 7-oxo-6-(sulfooxy)-, monosodium salt ("NXL104") as a diagnostic reagent for the detection of classes A and C-type beta-lactamases including carbapenemases expressed by *Enterobacteriaceae* and non-Enterobacteriaceae.

Diagnostic method for the detection of beta-lactamases as expressed by *Enterobacteriaceae* and non-Enterobacteriaceae

## Description

The compound (1R,2S,5R) 1,6-Diazabicyclo[3.2.1]octane-2-carboxamide, 7-oxo-6-(sulfooxy)-, monosodium salt (hereafter "NXL104") is a non-beta-lactam covalent inhibitor of a broad spectrum of serine beta-lactamases, described in application WO 02/10172. Its use as beta-lactamases inhibitor has made the subject of application WO 03/063684.

The structure of NXL104 is the following:

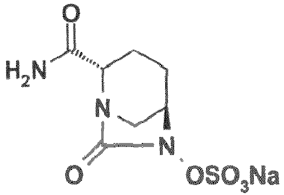

As mentioned above, NXL104 inhibits a broad spectrum of beta-lactamases enzymes, and this includes those belonging to Ambler classes A and C, and in particular KPC (*Klebsiella pneumoniae* carbapenemases), SME, NMC/IMI, GES and SFC families.

Carbapenemases are capable of hydrolyzing carbapenem type antibiotics. Bacteria expressing these enzymes are therefore frequently resistant to the carbapenems.

The KPC family is of particular concern as it has been reported in several occasions from *Klebsiella* and *Enterobacter* spp. from different places of the world.

The KPC beta-lactamases produced by *Enterobacteriaceae* and non-Enterobacteriaceae species can confer resistance to all beta-lactam agents including carbapenems. A major concern, as reported in Journal of Clinical Microbiology May 2007 p 2723-2725, is that so far no method has demonstrated 100% specificity for detection of carbapenemase activity.

Consequently, this type of resistance is difficult to detect in the routine clinical microbiology laboratories and therefore a real need exists to dispose of an efficient diagnostic method.

The applicant has discovered and developed a novel efficient method which uses NXL104 as a diagnostic reagent for the detection of classes A and C-type beta-lactamases including carbapenemases expressed by *Enterobacteriaceae* and non-Enterobacteriaceae and this novel use of NXL104 constitutes in itself a first subject of the invention.

In particular, an object of the invention is the use of NXL104 as a diagnostic reagent for the detection of carbapenemases-type beta-lactamases belonging to the families KPC, IMI, GES, SME, SFC and NMC expressed by *Enterobacteriaceae* and non-Enterobacteriaceae.

More particularly, an object of the invention is the use of NXL104 as a diagnostic reagent for the detection of carbapenemases-type beta-lactamases belonging to the families KPC-2, -3, -4, and also IMI -1, -2, GES -2, -4, -5, -6, SME -1, SFC -1, and NMC -A, expressed by *Enterobacteriaceae* and non-Enterobacteriaceae.

The use of NXL104 as a diagnostic reagent involves either the use of a simple set of absorbent supports, and preferably of paper disks in a number of formats, the use of NXL104 as part of a paper strip (E-test) or its use in liquid or agar medium for the detection of beta-lactamases expressed by bacteria.

Another object of the invention is a diagnostic method for the detection of beta-lactamases as expressed by *Enterobacteriaceae* and non-Enterobacteriaceae, characterized in that it comprises:
a- inoculating agar plates with a lawn of test *Enterobacteriaceae* or non-Enterobacteriaceae strain,
b- placing a first absorbent support containing 5-75 µg of a beta-lactam antibiotic alone on the agar plate,
c- placing another absorbent support, impregnated with NXL104 associated with the same amount as mentioned above of the beta-lactam, at an appropriate distance of 7.5 to 25 mm from the first beta-lactam-impregnated support,
d- visually evaluating the presence of expressed beta-lactamases as the expansion of the synergy zone between the beta-lactam and beta-lactam-NXL104 absorbent supports.

As shown below in the experimental part, when the bacterial strain in question expresses beta-lactamases the inhibition zone in the presence of the combination is significantly larger.

Another object of the invention is a diagnostic method for the detection of carbapenemases-type beta-lactamases as expressed by *Enterobacteriaceae* and non-Enterobacteriaceae, characterized in that it comprises:
a- inoculating agar plates with a lawn of test *Enterobacteriaceae* or non-*Enterobacteriaceae* strain,
b- placing a first absorbent support containing 5-75 µg of a carbapenem on the agar plate,
c- placing another absorbent support impregnated with NXL104 associated with the same amount as mentioned above of the carbapenem at an appropriate distance of 7.5 to 25 mm from the first carbapenem-impregnated support,
d- visually evaluating the presence of expressed carbapenemases as the expansion of the synergy zone between the carbapenem and carbapenem-NXL104 absorbent supports.

As shown below in the experimental part, when the bacterial strain in question expresses carbapenemases the inhibition zone in the presence of the combination is significantly larger.

In the two above diagnostic methods, preferably the absorbent support is impregnated with NXL104 in an amount of 1 to 1000 µg and the absorbent supports are paper disks.

Another object of the invention is a diagnostic method for the detection of beta-lactamases as expressed by *Enterobacteriaceae* and non-Enterobacteriaceae, characterized in that it comprises:
a- inoculating agar plates with a lawn of test *Enterobacteriaceae* or non-Enterobacteriaceae strain,
b- placing a first absorbent support strip impregnated with a concentration gradient of NXL104 on the agar plate,
c- placing another absorbent support strip, impregnated with a concentration gradient of a beta-lactam, at 7.5-25mm from the NXL 104-impregnated first support strip,
d- visually evaluating the presence of expressed beta-lactamases as the presence of a zone of synergy between the two absorbent supports strips as compared to the zone on the opposite side of the beta-lactam strip.

Another object of the invention is a diagnostic method for the detection of carbapenemases as expressed by *Enterobacteriaceae* and non-Enterobacteriaceae, characterized in that it comprises:
a- inoculating agar plates with a lawn of test *Enterobacteriaceae* or non-Enterobacteriaceae strain,
b- placing a first absorbent support strip impregnated with a concentration gradient of NXL104 on the agar plate,
c- placing another absorbent support strip, impregnated with a concentration gradient of a carbapenem, at 7.5-25mm from the NXL104-impregnated first support strip,
d- visually evaluating the presence of expressed carbapenemases as the presence of a zone of synergy between the two absorbent supports strips as compared to the zone on the opposite side of the carbapenem strip.

In the two above diagnostic methods, preferably the first absorbent support strip is impregnated with NXL104 in a decreasing concentration from 16µg to 1µg and the other absorbent support strip is impregnated with beta-lactams or carbapenems in a decreasing concentration from 32µg to 0.25µg, and the absorbent supports are made of paper strips.

Another object of the invention is a diagnostic method for the detection of beta-lactamases or carbapenemases-type beta-lactamases as expressed by *Enterobacteriaceae* and non-Enterobacteriaceae, characterized in that it comprises:
a- inoculating agar plates with a lawn of test *Enterobacteriaceae* or non-Enterobacteriaceae strain,
b- impregnating a half of an absorbent support strip with a constant concentration of NXL104 plus with a concentration gradient of either a beta-lactam or a carbapenem,
c- impregnating the other part of the absorbent support strip only with the same concentration gradient of either the beta-lactam or the carbapenem down the support strip,
d- visually evaluating the presence of expressed beta-lactamases or carbapenemases as the decrease in the point where the inhibition zone joins the part of the strip impregnated with NXL104.

Preferably, the absorbent support strip is impregnated with NXL104 in an amount from 64 µg to 1 µg and with beta-lactam or carbapenem in a decreasing amount from 32µg to 0.25µg.

Preferably, the absorbent supports are made of paper strips.

In general, the technique using the absorbent support strip referred to in the above description, known as Etest®, and the interpretation of the results has been described *inter alia* in the following references:
J Clin Microbiol, 34(8), 1880-1884, 1996; ICAAC 1999 poster 898; J Antimicrob Chemotherap, 54, 134-138, 2004.

Another object of the invention is a method using liquid MIC test according to CLSI (Clinical Laboratory Standard Institute, formerly, NCCLS) guidelines (CLSI M7-A7, January 2006, "Methods for dilution antimicrobial susceptibility tests for bacteria that grow aerobically") for the beta-lactam of interest.

In the case of testing for beta-lactamases including carbapenemases according to the invention, a beta-lactam or carbapenem is diluted in two fold steps from 32 to 0.25 µg/ml in the presence or absence of 1 to 16 µg/ml of NXL104, the presence of expressed beta-lactamases including carbapenemases is confirmed by the greater than four fold improved susceptibility to the beta-lactam or carbapenem in the presence of NXL104.

Preferably the NXL104 is present in an amount of 4 µg/ml.

In another example of a liquid based diagnostic test, bacterial growth rates are measured in the presence of specified concentrations of beta-lactams including carbapenems in the presence and absence of 1-16 µg/ml NXL104.

Another object of the invention includes methods of treating an antibiotic resistant patient by administering NXL104 in combination with an antibiotic. The patient may be treated before or after the detection of a beta-lactamase or carbapenemase-type beta-lactamase. For example, beta-lactamases including carbapenemases as expressed by *Enterobacteriaceae* and non-Enterobacteriaceae.

Another object of the invention includes methods of treating a patient infected with a bacterium expressing a beta-lactamase or carbapenemase-type beta-lactamase by administering NXL104 in combination with an antibiotic. For example, beta-lactamases including carbapenemases as expressed by *Enterobacteriaceae* and non-Enterobacteriaceae. The infection of the patient with a bacterium expressing a beta-lactamase or carbapenemase-type beta-lactamase may be determined before or after treatment with the combination of NXL104 and an antibiotic.

In some embodiments, the treatments may include administering NXL104 in combination with an antibiotic chosen from the group consisting of the penams, the penems, the carbapenems, the cephems, the cephalosporins, the carbacephems, the oxacephems, the cephamycins and the monobactams. In exemplary embodiments, the treatments may include administering NXL104 in combination with amoxicillin, ampicillin, azlocillin, mezlocillin, apalcillin, hetacillin, bacampicillin, carbenicillin, sulbenicillin, ticarcillin, piperacillin, azlocillin, mecillinam, pivmecillinam, methicillin, ciclacillin, talampicillin, aspoxicillin, oxacillin, cloxacillin, dicloxacillin, flucloxacillin, nafcillin, pivampicillin, cephalothin, cephaloridine, cefaclor, cefadroxil, cefamandole, cefazolin, cephalexin, cephradine, ceftaroline, ceftizoxime, cefoxitin, cephacetrile, cefotiam, cefotaxime, cefsulodin, cefoperazone, ceftizoxime, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidin, cefixime, ceftibuten, cefdinir, cefpodoxime axetil, cefpodoxime proxetil, cefteram pivoxil, cefetamet pivoxil, cefcapene pivoxil cefditoren pivoxil, cefuroxime, cefuroxime axetil, loracarbacef, latamoxef, imipenem, meropenem, biapenem, panipenem, aztreonam carumonam and pharmaceutically acceptable salts thereof.

A further object of the invention is a diagnostic kit for the detection of beta-lactamases including carbapenemases as expressed by *Enterobacteriaceae* and non-Enterobacteriaceae, characterized in that it comprises a microtitre plate support impregnated with 1 to 1000 µg of NXL104, alone or with 0.25 to 256 µg of a beta-lactam antibiotic.

In a particular embodiment, the absorbent support comprised in the diagnostic kit of the invention is a paper disk.

### Experimental part:

### Example 1:

General method: A 6.5 mm x 1mm thick paper disk is impregnated with NXL104 in an appropriate amount of from 1 to 1000 µg. The NXL104 may be combined with another antibiotic on the same disk or not. A lawn of bacteria is inoculated onto an agar plate. The NXL104 containing disk is placed at an appropriate distance of from 7.5 to 25 mm from a disk impregnated with a carbapenem (e.g. meropenem, ertapenem, imipenem, doripenem, or others). As shown in the figures below, bacteria resistant to the carbapenems by virtue of the appropriate expressed beta-lactamase will show a zone of synergy between the two disks.
1. Bacterial isolates: A control KPC negative strain, *K. pneumoniae* ATCC 700603 (SHV-18 ESBL), and three KPC positive clinical isolates, *K. pneumoniae* 283 KB7 (KPC-2), *E. coli* 250 SuJ1 (KPC-2), *E. cloacae* MAC (KPC-3, TEM-1, OXA-9) were tested for the detection of the carbapenemase enzyme.
2. MIC testing: as per CLSI microbroth doubling dilution technique with penicillin, cephalosporins, and imipenem alone and with a fixed concentration of 4µg/ml NXL104.
3. Detection of carbapenamase: Mueller-Hinton agar plates (150mm plastic Petri dishes) were inoculated with a lawn of 0.5 McFarland (∼10⁸ cfu/mL) adjusted test strains. A 10µg imipenem (IPM) disk was placed at a distance of 20-22mm apart from a disk of 30µg Ceftazidime/60µg NXL104 (CAZ/104). Plates were inverted and incubated at 37°C overnight.

Synergy is seen as an expansion of the imipenem zone adjacent to the NXL104 containing disk. The following figures demonstrate the absence or presence of the KPC carbapenamase in the above-mentioned strains:
(CAZ = Ceftazidime 30µg; CAZ104 = Ceftazidime 30µg+NXL104-60µg; IPM = Imipenem 10µg)

### Double Disk Synergy Test for detection of KPC β-lactamases

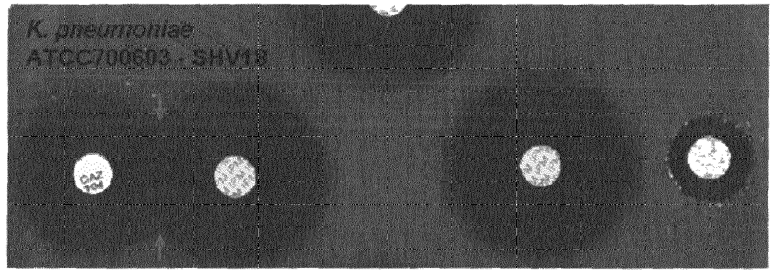
[?]
[?]

MICs:
CAZ - 64µg/mL; IPM - 0.5µg/mL; CAZ/NXL - 2µg/mL
Control KPC negative *K*. *pneumoniae* ATCC 700603 strain: additive effect (shown by green arrows) and not expansion of the synergy zone 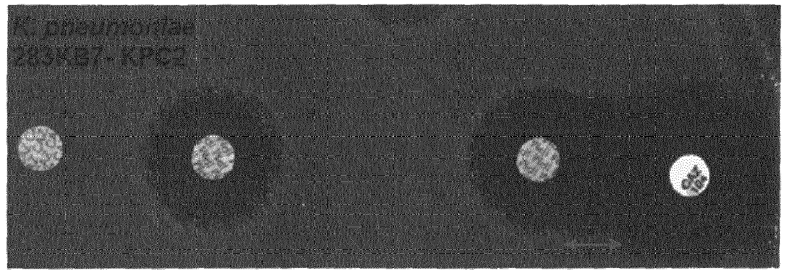 MICs:
CAZ ->128µg/mL; IPM - 32µg/mL; CAZ/NXL - 2µg/mL; IPM/NXL - 0.125µg/mL
Green arrows: expansion of the synergistic zone
KPC positive *K. pneumoniae* strain: expansion of the synergy zone (shown by green arrows) 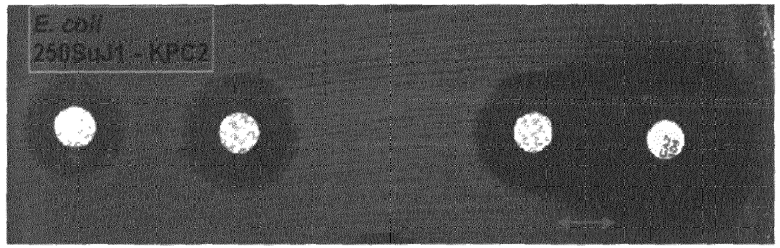
[?]
[?]
 MICs:
CAZ ->128µg/mL; IPM - 16µg/mL; CAZ/NXL - 0.5µg/mL; IPM/NXL - 0.25µg/mL
Green arrow: expansion of the synergistic zone
KPC positive *E. coli* strain: expansion of the synergy zone (shown by green arrows) 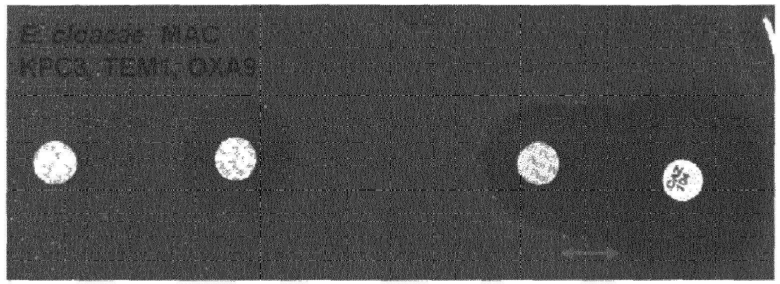 MICs:
CAZ ->128µg/mL; IPM - 64µg/mL; CAZ/NXL - 4µg/mL; IPM/NXL - 2µg/mL
Green arrow: expansion of the synergistic zone
KPC positive *E. cloacae* strain: expansion of the synergy zone (shown by green arrows)

### Example 2:

In another application, a commercially available disk containing a carbapenem alone and the same carbapenem disk containing NXL104 between 1 and 64 µg are placed on a lawn of bacteria on agar. An increase in the zone of inhibition in the presence of NXL104 indicates the presence of a class A carbapenem hydrolyzing beta-lactamase.

### Example 3:

In the paper strip method, a decreasing amount of NXL104 from 64-1 µg impregnates a paper strip, which is then placed near a similar strip with a decreasing amount of a carbapenem from 32 to 0.25 µg. A zone of synergy will be seen with strains expressing a class A carbapenem hydrolyzing beta-lactamase. In another context, a strip bearing a constant amount usually 16 µg of NXL104 plus a carbapenem in decreasing amounts from 32 to 0.25 µg down the strip is used along with a strip impregnated only with a decreasing amount as noted of the carbapenem. A decrease in the point where the inhibition zone joins the strip in the presence of NXL104 indicates the presence of a class A carbapenem hydrolyzing beta-lactamase.

### Example 4:

For applications in liquid or agar media, a carbapenem antibiotic is tested while diluted in the absence and separately in the presence of NXL104. A decrease in the carbapenem concentration required to inhibit the growth of the bacteria in the presence of NXL104 compared to its absence indicates the presence of a class A carbapenem hydrolyzing beta-lactamase.

### General method:

1) Bacteria were grown overnight in cation adjusted Mueller-Hinton (MH) broth at 37°C. A 1/10 dilution of the overnight culture was seeded in prewarmed MH broth and optical density measured and adjusted to McFarland standard 0.5 to obtain ∼10⁸ CFU/mL (Colony Forming Units). Bacterial inoculum was prepared from this culture and diluted to achieve the desired starting inoculum of ∼5x10⁵ CFU/mL.
2) Drug combinations:beta-lactam antibiotics were used alone, and in combinations with the beta-lactamase inhibitor at a fixed concentration of 4µg/mL.
3) MIC determination

A 100µL volume of the bacterial suspension was added to the 96 well flat-bottom (NUNC) microtitre plates containing a 100µL volume of the diluted beta-lactam antibiotic alone and with the beta-lactamase inhibitor. Plates were incubated at 37°C overnight. The quality control strains included *K. pneumoniae* ATCC 700603 strain with SHV-18 beta-lactamase and a fully susceptible *E. coli* ATCC 25922 strain.

### 4) Observations and measurements

Minimum inhibitory concentrations for CAZ, PIP, CTX, and IPM alone and with 4 µg/mL concentration of NXL104 were determined using CLSI (formerly the NCCLS) methods for antimicrobial susceptibility testing with Mueller-Hinton (MH) broth. MIC was defined as the lowest concentration that inhibited all visual growth. The *in vitro* antibacterial activity was analysed for restoration of MICs.
(CAZ = ceftazidime; PIP= piperacillin, CTX = cefotaxime, IPM = imipenem)

### 5) Results

*Enterobacteriaceae* with class A TEM, SHV, CTX-M, extended spectrum beta-lactamases (ESBL) and class C enzymes:
Results are shown in Tables 1 and 2.

Potentiation of cefotaxime by NXL104 was generally >1000-fold for isolates with enzymes belonging to any CTX-M group with MICs reduced from >128 µg/mL to <0.125 to 0.25 µg/mL. MICs of ceftazidime for these isolates were reduced from >128 µg/mL to <0.125 to 4 µg/mL. MICs were also reduced for piperacillin with NXL104 from >128 µg/mL to 2 - 16 µg/mL for all the CTX-M group isolates. Imipenem was very active both alone and with NXL104 combination with MICs of 0.25 - 2 µg/mL and <0.125 to 2 µg/mL respectively.

MICs of ceftazidime and piperacillin were reduced by NXL104 for other class A ESBLs (SHV, TEM, non classical class A) from >128 µg/mL to 1 - 8 µg/mL and to 4 to 16 µg/mL respectively. As with CTX-M groups, imipenem was very active both alone and with NXL104 combination with MICs of 0.25 - 4 µg/mL and <0.125 to 1 µg/mL respectively.

For isolates with the class C enzymes (including plasmid - borne and chromosomal enzymes), potentiation of ceftazidime by NXL104 was >256-fold and ceftazidime MICs were reduced from 32 - >128 µg/mL to <0.125 to 2. The piperacillin MICs were also reduced with NXL104 combination from >128 µg/mL to 1 - 16 µg/mL. Imipenem was active both alone and with NXL104 combination.

Against the two isolates with metallo enzymes (VIM-1, -4), the MICs of none of the β-lactam antibiotics with NXL104 were reduced.

Overall, potentiation of ceftazidime by NXL104 was very good for the ceftazidime-resistant isolates with MICs reduced from 32 - >128 to <0.125 to 8 in 28 out of 33 (85%) ceftazidime-resistant isolates. MIC of piperacillin was reduced from >128 µg/mL to 1 - 16 µg/mL with NXL 104 in 30/33 strains. Against these resistant isolates, imipenem both alone and in combination with NXL104 was very active with low MICs.

*Enterobacteriaceae* with class A carbapenemases (KPC-2 and KPC-3):
Results for isolates with KPC enzymes are shown in Table 3.

NXL104 reduced dramatically the MICs of imipenem from 8 - 64 µg/mL to <0.125 - 2 µg/mL.

NXL104 reduced the MICs of ceftazidime from 32 - >128 µg/mL to 0.5 - 8 µg/mL.

Although only 4/10 isolates were tested with cefotaxime, NXL104 reduced the cefotaxime MICs from 32 -128 µg/mL to 0.25 to 2 µg/mL.

While not as impressive as for ceftazidime, imipenem, and cefotaxime, NXL104 also reduced the piperacillin, MICs from >128 µg/mL to 1 - 16 µg/mL.

In resumé:
The activity of CAZ and PIP with NXL104 combinations was highly potentiated against 28/33 (∼85%) CAZ-resistant clinical *enterobacteriaceae* isolates with:
   Class A TEM enzymes, including ESBL
   Class A SHV enzymes, including ESBL
   Class A CTX-M enzymes, including CAZ-hydrolyzing enzymes
   Non classical class A enzymes (VEB, PER)
   Class C enzymes, including plasmid-borne and chromosomal enzymes

NXL104 also potentiated the inhibitory activity of CTX although not all strains were tested with CTX/NXL104 combination.

IPM was the most active agent against these clinical isolates.

The activity of CAZ, PIP, and IPM with NXL104 combinations was highly potentiated against these clinical isolates with Class A KPC carbapenemases.

These above findings demonstrate the use of NXL104 as a diagnostic reagent for the detection of beta-lactamases including carbapenemases.

**Table 1. MICs (µg/mL) for clinical isolates with various beta-lactamases**

| **Strain ID** | **Organism** | **Mechanism** | **CAZ** | | **IPM** | | **PIP** | | **CTX** | |
|---|---|---|---|---|---|---|---|---|---|---|
| | | | **alone** | **+NXL104** | **alone** | **+NXL104** | **alone** | **+HXL104** | **alone** | **+NXL104** |
| KP02 | *K. pneumoniae* | CTX-M-1 | 2 | ≤0.125 | 0.5 | 0.5 | ≥128 | 2 | 32 | ≤0.125 |
| SCI | *E. coli* | CTX-M-1, TEM-1 | 0 | 0.25 | 0.25 | ≤0.125 | ≥128 | 4 | ≥128 | ≤0.125 |
| TN06 | *E. coli* | CTX-M-2, TEM-1 | 32 | ≤0.125 | 0.25 | ≤0.125 | ≥128 | 1 | ≥128 | ≤0.125 |
| 465 | *K. pneumoniae* | CTX-M-2, TEM-18 | 64 | 4 | 1 | 1 | ≥128 | 16 | nd | nd |
| 238 | *K. pneumoniae* | CTX-M-2,SHV-2, TEM-12 | ≥128 | 2 | 0.25 | 0.5 | ≥128 | 8 | nd | nd |
| 253 | *K. pneumoniae* | CTX-M-2, SHV-5, TEM-12 | ≥128 | 1 | 0.5 | 1 | ≥128 | 8 | nd | nd |
| KP-LT | *K. pneumoniae* | CTX-M-3 | 8 | 1 | 0.25 | 0.25 | ≥128 | 16 | ≥128 | 0.25 |
| SM01 | *S. marcescens* | CTX-M-3, TEM-1 | 4 | 0.25 | 1 | 0.5 | ≥128 | 2 | ≥128 | ≤0.125 |
| 427 | *K. pneumoniae* | CTX-M-3,SHV-1, TEM-18 | ≥128 | 1 | 0.5 | 1 | ≥128 | 16 | nd | nd |
| TN05 | *E. coli* | CTX-M-9 | 1 | 0.25 | 0.25 | ≤0.125 | ≥128 | 4 | ≥128 | ≤0.125 |
| KP04 | *K. pneumoniae* | CTX-M-14 | 32 | 1 | 2 | 2 | ≥128 | 8 | ≥128 | 0.25 |
| TN07 | *E. coli* | CTX-M-14 | 2 | 0.25 | 0.25 | ≤0.125 | ≥128 | 2 | ≥128 | ≤0.125 |
| TN13 | *E. coli* | CTX-M-14, CMY-2, TEM-1 | 128 | 0.5 | 0.5 | ≤0.125 | ≥128 | 4 | ≥128 | 0.25 |
| TN03 | *E. coli* | CTX-M-15, TEM-1, OXA-1 | 128 | 0.5 | 0.25 | ≤0.125 | ≥128 | 4 | ≥128 | ≤0.125 |
| Tunisie clone K4 | *K. pneumoniae* | CTX-M-15, TEM-1, OXA-1 | ≥128 | 1 | 0.25 | ≤0.125 | ≥128 | 16 | ≥128 | ≤0.125 |
| Tunisie clone E4 | *E. coli* | CTX-M-16, TEM-1 | 128 | 1 | 0.25 | 0.25 | ≥128 | 4 | ≥128 | 0.25 |
| Tunisie clone K1 | *K. pneumoniae* | CTX-M-16, OXA-1 | ≥128 | 2 | 1 | 1 | ≥128 | 8 | ≥128 | 0.25 |
| 441 | *K. pneumoniae* | SHV-1, TEM-2, PER | ≥128 | 8 | 0.25 | 0.5 | ≥128 | 8 | nd | nd |
| 449 | *K. pneumoniae* | SHV-1, TEM-2, PER | ≥128 | 8 | 0.25 | 0.5 | ≥128 | 16 | nd | nd |
| 60 | *K. pneumoniae* | SHV-2, TEM-2, PER | 64 | 4 | 0.25 | 0.5 | ≥128 | 8 | nd | nd |
| 26 | *K. pneumoniae* | SHV-5 | ≥128 | 0.5 | 0.5 | 0.25 | ≥128 | 8 | nd | nd |
| 444 | *K. pneumoniae* | SHV-5, TEM-2, PER | ≥128 | 16 | 0.5 | 0.5 | ≥128 | 16 | nd | nd |
| 236 | *K. pneumoniae* | SHV-5, TEM-10 | ≥128 | 2 | 2 | 1 | ≥128 | 4 | nd | nd |
| 243 | *K. pneumoniae* | SHV-5, TEM-63 | ≥128 | 1 | 1 | 1 | ≥128 | 8 | nd | nd |
| 181 | *K. pneumoniae* | SHV-5, TEM-10 | ≥128 | 2 | 4 | 1 | ≥128 | 16 | nd | nd |
| 157 | *K. pneumoniae* | SHV-5, TEM-26 | ≥128 | 1 | 0.25 | 0.25 | ≥128 | 8 | nd | nd |
| 1547 | *K. oxytoca* | OXY-2, TEM-1 | 4 | 1 | 0.5 | 0.5 | ≥128 | 8 | nd | nd |
| 16944 | *K. oxytoca* | OXY-2, TEM-1 | ≥128 | 2 | 0.5 | 0.5 | ≥128 | 8 | nd | nd |
| IND | *E. coli* | VEB-1 + CMY-2 | ≥128 | 64 | 1 | 0.25 | ≥128 | 8 | nd | nd |
| 1431 | *K. oxytoca* | TEM-129 | ≥128 | 2 | 0.25 | 0.25 | ≥128 | 8 | nd | nd |
| EAR2 | *E. aerogenes* | Case R FEP | ≥128 | 2 | 1 | ≤0.125 | ≥128 | 8 | nd | nd |
| CHE | *E. cloacae* | Case R FEP | ≥128 | 32 | 0.5 | 0.5 | ≥128 | 32 | nd | nd |
| KOL | *K. pneumoniae* | MOX-2, SHV-5, TEM-1 | ≥128 | 1 | 0.25 | ≤0.125 | ≥128 | 16 | nd | nd |
| 1734 | *K. pneumoniae* | FOX-3, TEM-1 | 128 | 2 | 0.5 | 0.25 | 128 | 16 | nd | nd |
| SLK54 | *K. pneumoniae* | ACC-1 + TEM-1 | 128 | 1 | 0.5 | ≤0.125 | ≥128 | 8 | nd | nd |
| H223b | *P. mirabilis* | CMY-4 | 32 | ≤0.125 | 4 | 2 | ≥128 | 1 | nd | nd |
| 9701 | *K. pneumoniae* | CMY-4 + TEM-1 | 128 | 0.5 | 1 | 1 | ≥128 | 8 | nd | nd |
| TN58467 | *K. pneumoniae* | DHA-1 + SHV-2a + TEM-1 | ≥128 | 1 | 0.5 | ≤0.125 | ≥128 | 16 | nd | nd |
| MPDHA | *K. oxytoca* | DHA-1 + TEM-1 | 2 | ≤0.125 | 1 | ≤0.125 | ≥128 | 2 | nd | nd |
| strain patient F | *K. pneumoniae* | VIM-1 + SHV-5 | ≥128 | ≥128 | 32 | 32 | ≥128 | ≥128 | nd | nd |
| Patient 1 BHR | *K. pneumoniae* | VIM-4 + CTX-M-15 + CMY-4 + TEM-1 | ≥128 | 16 | 16 | 8 | ≥128 | ≥128 | ≥128 | ≥128 |

| | | | | | | | | | | |
|---|---|---|---|---|---|---|---|---|---|---|
| nd: not done | | | | | | | | | | |

**Table 2. MICs (µg/mL) for ceftazidime-resistant clinical isolates with various beta-lactamases**

| **Strain ID** | **Organism** | **Mechanism** | **CAZ** | | **IPM** | | **PIP** | | **CTX** | |
|---|---|---|---|---|---|---|---|---|---|---|
| | | | **alone** | **+NXL 104** | **alone** | **+NXL104** | **alone** | **+NXL 104** | **alone** | **+NXL 104** |
| 238 | *K. pneumoniae* | CTX-M-2,SHV-2, TEM-12 | ≥128 | 2 | 0.25 | 0.5 | ≥128 | 8 | nd | nd |
| 253 | *K. pneumoniae* | CTX-M-2, SHV-5, TEM-12 | ≥128 | 1 | 0.5 | 1 | ≥128 | 8 | nd | nd |
| 427 | *K. pneumoniae* | CTX-M-3,SHV-1, TEM-1B | ≥128 | 1 | 0.5 | 1 | ≥128 | 16 | nd | nd |
| Tunisie clone K4 | *K. pneumoniae* | CTX-M-15, TEM-1, OXA-1 | ≥128 | 1 | 0.25 | 0.25 | ≥128 | 16 | ≥128 | ≤0.125 |
| Tunisie clone K1 | *K. pneumoniae* | CTX-M-16, OXA-1 | ≥128 | 2 | 1 | 1 | ≥128 | 8 | ≥128 | 0.25 |
| 441 | *K. pneumoniae* | SHV-1, TEM-2, PER | ≥128 | 8 | 0.25 | 0.5 | ≥128 | 8 | nd | nd |
| 449 | *K. pneumoniae* | SHV-1, TEM-2, PER | ≥128 | 8 | 0.25 | 0.5 | ≥128 | 16 | nd | nd |
| 26 | *K. pneumoniae* | SHV-5 | ≥128 | 0.5 | 0.5 | 0.25 | ≥128 | 8 | nd | nd |
| 444 | *K. pneumoniae* | SHV-5, TEM-2, PER | ≥128 | 16 | 0.5 | 0.5 | ≥128 | 16 | nd | nd |
| 236 | *K. pneumoniae* | SHV-5, TEM10 | ≥128 | 2 | 2 | 1 | ≥128 | 4 | nd | nd |
| 243 | *K. pneumoniae* | SHV-5, TEM-63 | ≥128 | 1 | 1 | 1 | ≥128 | 8 | nd | nd |
| 181 | *K. pneumoniae* | SHV-5, TEM-10 | ≥128 | 2 | 4 | 1 | ≥128 | 16 | nd | nd |
| 157 | *K. pneumoniae* | SHV-5, TEM-26 | ≥128 | 1 | 0.25 | 0.25 | ≥128 | 8 | nd | nd |
| 16944 | *K. oxytoca* | OXY-2, TEM-1 | ≥128 | 2 | 0.5 | 0.5 | ≥128 | 8 | nd | nd |
| IND | *E*. *coli* | VEB-1 + CMY-2 | ≥128 | 64 | 1 | 0.25 | ≥128 | 8 | nd | nd |
| 1431 | *K. oxytoca* | TEM-129 | ≥128 | 2 | 0.25 | ≤0.125 | ≥128 | 8 | nd | nd |
| EAR2 | *E. aerogenes* | Case R FEP | ≥128 | 2 | 1 | 0.5 | ≥128 | 8 | nd | nd |
| CHE | *E. cloacae* | Case R FEP | ≥128 | 32 | 0.5 | 0.5 | ≥128 | 32 | nd | nd |
| TN58467 | *K. pneumoniae* | DHA-1 + SHV-2a + TEM-1 | ≥128 | 1 | 0.5 | ≤0.125 | ≥128 | 16 | nd | nd |
| strain patient F | *K. pneumonia* | VIM-1 + SHV-5 | ≥128 | ≥128 | 32 | 32 | ≥128 | ≥128 | nd | nd |
| Patient 1 BHR | *K. pneumoniae* | VIM -4 + CTX-M-15 + CMY-4 + TEM-1 | ≥128 | 16 | 16 | 8 | ≥128 | ≥128 | ≥128 | ≥128 |
| TN13 | *E. coli* | CTX-M-14, CMY-2, TEM-1 | 128 | 0.5 | 0.5 | ≤0.125 | ≥128 | 4 | ≥128 | 0.25 |
| TN03 | *E. coli* | CTX-M-15, TEM-1, OXA-1 | 128 | 0.5 | 0.25 | ≤0.125 | ≥128 | 4 | ≥128 | ≤0.125 |
| Tunisie clone E4 | *E. coli* | CTX-M-16, TEM1 | 128 | 1 | 0.25 | 0.25 | ≥128 | 4 | ≥128 | 0.25 |
| KOL | *K. pneumoniae* | MOX-2, SHV-5, TEM-1 | 128 | 1 | 0.25 | ≤0.125 | ≥128 | 16 | nd | nd |
| 1734 | *K. pneumoniae* | FOX-3, TEM-1 | 128 | 2 | 0.5 | 0.25 | ≥128 | 16 | nd | nd |
| SLK54 | *K. pneumoniae* | ACC-1 + TEM-1 | 128 | 1 | 0.5 | ≤0.125 | ≥128 | 8 | nd | nd |
| 9701 | *K. pneumoniae* | CMY-4 + TEM-1 | 128 | 0.5 | 1 | 1 | ≥128 | 8 | nd | nd |
| 465 | *K. pneumoniae* | CTX-M-2, TEM-1B | 64 | 4 | 1 | 1 | ≥128 | 16 | nd | nd |
| 60 | *K. pneumoniae* | SHV-2, TEM-2, PER | 64 | 4 | 0.25 | 0.5 | ≥128 | 8 | nd | nd |
| TNO6 | *E. coli* | CTX-M-2, TEM-1 | 32 | ≤0.125 | 0.25 | ≤0.125 | ≥128 | 1 | ≥128 | ≤0.125 |
| KPO4 | *K. pneumoniae* | CTX-M-14 | 32 | 1 | 2 | 2 | ≥128 | 8 | ≥128 | 0.25 |
| H223b | *P. mirabilis* | CMY-4 | 32 | ≤0.125 | 4 | 2 | ≥128 | 1 | nd | nd |
| SCI | *E. coli* | CTX-M-1, TEM-1 | 8 | 0.25 | 0.25 | ≤0.125 | ≥128 | 4 | ≥128 | ≤0.125 |
| KP-LT | *K. pneumoniae* | CTX-M-3 | 8 | 1 | 0.25 | 0.25 | ≥128 | 16 | ≥128 | 0.25 |
| SMO1 | S. *marcescens* | CTX-M-3, TEM-1 | 4 | 0.25 | 1 | 0.5 | ≥128 | 2 | ≥128 | ≤0.125 |
| 1547 | *K. oxytoca* | OXY-2, TEM-1 | 4 | 1 | 0.5 | 0.5 | ≥128 | 8 | nd | nd |
| KP02 | *K. pneumoniae* | CTX-M-1 | 2 | ≤0.125 | 0.5 | 0.5 | ≥128 | 2 | 32 | ≤0.125 |
| TN07 | *E. coli* | CTX-M-14 | 2 | 0.25 | 0.25 | ≤0.125 | ≥128 | 2 | ≥128 | ≤0.125 |
| MPDHA | *K. oxytoca* | DHA-1 + TEM-1 | 2 | ≤0.125 | 1 | ≤0.125 | ≥128 | 2 | nd | nd |
| TN05 | *E. coli* | CTX-M-9 | 1 | 0.25 | 0.25 | ≤0.125 | ≥128 | 4 | ≥128 | ≤0.125 |

| | | | | | | | | | | |
|---|---|---|---|---|---|---|---|---|---|---|
| The black line demarcates the CAZ -resistant strains from the -susceptible strains nd: not done | | | | | | | | | | |

**Table 3. MICs (µg/mL) for clinical isolates with class A carbapenemases**

| **Strain** ID | **Organism** | **Mechanism** | **CAZ** | | **IPM** | | **PIP** | |
|---|---|---|---|---|---|---|---|---|
| | | | **alone** | **+NXL104** | **alone** | **+NXL104** | **alone** | **+NXL104** |
| 2138 | *E*. *coli* | KPC-2 | 32 | 0.5 | 32 | 0.25 | ≥128 | 4 |
| YC | *K. pneumoniae* | KPC-2 | ≥128 | 1 | 84 | ≤0.125 | ≥128 | 16 |
| 7506 | *E*. *cloacae* | KPC-2 | ≥128 | 4 | 16 | 0.5 | ≥128 | 32 |
| VAKP | *K. pneumoniae* | KPC-2 | 128 | 1 | 32 | 2 | ≥128 | 16 |
| VA8 | *K. pneumoniae* | KPC-3 | ≥128 | 1 | 0.25 | 0.25 | ≥128 | 8 |
| MAC | *E. cloacae* | KPC-3, TEM-1, OXA-9 | ≥128 | 4 | 64 | 0.5 | ≥128 | 16 |

### Example 5:

Kits have been prepared as a 96 well flat-bottom (NUNC) microtitre plate which contains a 100µL volume of the diluted beta-lactam antibiotic alone or the combination of beta-lactam and the beta-lactamase inhibitor NXL104.Kits have been formatted such as in Microscan microtitre plates (liquid MIC method above), Vitek (bacterial growth rate comparison above) and others based roughly on these two.

## Claims

1. Use of (1R,2S,5R) 1,6-diazabicyclo[3.2.1]octanc-2-carboxamide, 7-oxo-6-(sulfooxy)-, monosodium salt ("NXL104") as a diagnostic reagent for the detection of classes A and C-type beta-lactamases including carbapenemases expressed by *Enterobacteriaceae* and non-Enterobacteriaceae.

2. Use according to claim 1, of (1R,2S,5R) 1,6-diazabicyclo[3.2.1]octane-2-carboxamide, 7-oxo-6-(sulfooxy)-, monosodium salt ("NXL104") as a diagnostic reagent for the detection of carbapenemases-type betalactamases belonging to the families KPC, IMI, GES, SME, SFC and NMC expressed by *Enterobacteriaceae* and non-Enterobacteriaceae.

3. Use according to claim 1 or 2, of (1R,2S,5R) 1,6-diazabicyclo[3.2.1]octane-2-carboxamide, 7-oxo-6-(sulfooxy)-, monosodium salt ("NXL104") as a diagnostic reagent for the detection of carbapenemases-type beta-lactamases belonging to the families KPC-2, -3, -4, IMI -1, -2, GES -2, -4, -5, -6, SME -1, SFC -1, and NMC -1 expressed by *Enterobacteriaceae* and non-Enterobacteriaceae.

4. Use according to any of claims 1 to 3, of (1R,2S,5R) 1,6-diazabicyclo[3.2.1]octane-2-carboxamide, 7-oxo-6-(sulfooxy)-, monosodium salt ("NXL104") as a diagnostic reagent for the detection of carbapenemases-type beta-lactamases belonging to the families KPC-2, -3, -4 expressed by *Enterobacteriaceae* and non-Enterobacteriaceae.

5. Diagnostic method for the detection of beta-lactamases as expressed by *Enterobacteriaceae* and non-Enterobacteriaceae, **characterized in that** it comprises:
a) inoculating agar plates with a lawn of test *Enterobacteriaceae* or non-Enterobacteriaceae strain,
b) placing a first absorbent support containing 5-75 µg of a beta-lactam antibiotic alone on the agar plate,
c) placing another absorbent support, impregnated with NXL104 associated with the same amount as above of the beta-lactam, at an appropriate distance of 7,5 to 25 mm from the first beta-lactam-impregnated support,
d) visually evaluating the presence of expressed beta-lactamases as the expansion of the synergy zone between the beta-lactam and beta-lactam-NXL 104 absorbent supports.

6. Diagnostic method for the detection of carbapenemases-type beta-lactamases as expressed by *Enterobacteriaceae* or non-Enterobacteriaceae strains, **characterized in that** it comprises:
a) inoculating agar plates with a lawn of test *Enterobacteriaceae* or non-Enterobacteriaceae strain,
b) placing a first absorbent support containing 5-75 µg of a carbapenem on the agar plate,
c) placing another absorbent support impregnated with NXL104 associated with the same amount as mentioned above of the carbapenem at an appropriate distance of 7,5 to 25 mm from the first carbapenem-impregnated support,
d) visually evaluating the presence of expressed carbapenemases as the expansion of the synergy zone between the cabapenem and carbapenem-NXL104 absorbent supports

7. Diagnostic method according to claim 5 or 6, **characterized in that** the absorbent support is impregnated with NXL 104 in an amount of 1 to 1000 µg.

8. Diagnostic method according to any of claims 5 to 7, **characterized in that** the absorbent support is a paper disk.

9. Diagnostic method for the detection of beta-lactamases as expressed by *Enterobacteriaceae* and non-Enterobacteriaceae, **characterized in that** it comprises:
a) inoculating agar plates with a lawn of test *Enterobacteriaceae* or non-Enterobacteriaceae strain,
b) placing a first absorbent support strip impregnated with a concentration gradient of NXL104 on the agar plate,
c) placing another absorbent support strip, impregnated with a concentration gradient of a beta-lactam, at 7.5-25mm from the NXL 104-impregnated first support strip,
d) visually evaluating the presence of expressed beta-lactamases as the presence of a zone of synergy between the two absorbent supports strips as compared to the zone on the opposite side of the beta-lactam strip.

10. Diagnostic method for the detection of carbapenemases as expressed by *Enterobacteriaceae* and non-Enterobacteriaceae, **characterized in that** it comprises:
a) inoculating agar plates with a lawn of test *Enterobacteriaceae* or non-Enterobacteriaceae strain,
b) placing a first absorbent support strip impregnated with a concentration gradient of NXL104 on the agar plate,
c) placing another absorbent support strip impregnated with a concentration gradient of a carbapenem at 7.5-25mm from the NXL104-impregnated first support strip,
d) visually evaluating the presence of carbapenemases as the presence of a zone of synergy between the two absorbent support strips as compared to the zone on the opposite side of the carbapenem strip.

11. Diagnostic method according to claim 9 or 10, **characterized in that** the first absorbent support strip is impregnated with NXL104 in a concentration gradient of from 16µg to 1µg. and the other absorbent support is impregnated with beta-lactam or carbapenem in a concentration gradient of from 32µg to 25µg.

12. Diagnostic method according to any of claims 9 to 11, **characterized in that** the absorbent support strip is paper strip.

13. Diagnostic method for the detection of beta-lactamases or carbapenemases-type beta-lactamases as expressed by *Enterobacteriaceae* and non-Enterobacteriaceae, **characterized in that** it comprises:
a) inoculating agar plates with a lawn of test *Enterobacteriaceae* or non-Enterobacteriaceae strain,
b) impregnating a half of an absorbent support strip with a constant concentration of NXL104 plus with a concentration gradient of either a beta-lactam or a carbapenem down the paper strip,
c) impregnating the other part of the absorbent support strip with the same concentration gradient of only either the beta-lactam or the carbapenem,
d) visually evaluating the presence of expressed beta-lactamases or carbapenemases as the decrease in the point where the inhibition zone joins the part of the strip impregnated with NXL 104.

14. Diagnostic method according to claim 13, **characterized in that** the support strip is impregnated with NXL104 in an amount of from 64µg to 1µg and with carbapenem in a decreasing amount of from 32µg to 0.25µg.

15. Diagnostic method according to claim 13 or 14, **characterized in that** the absorbent support strip is paper strip.

16. Diagnostic method for the detection of beta-lactamases or carbapenemases-type beta-lactamases as expressed by *Enterobacteriaceae* and non-Enterobacteriaceae, **characterized in that** it comprises diluting a carbapenem in two fold steps from 32 to 0.25 µg/ml in the presence or absence of 1 to 16 µg/ml of NXL104 and evaluating the presence of expressed beta-lactamases or carbapenemases by the greater than four fold improved susceptibility to the beta-lactam or carbapenem in the presence of NXL104.

17. Diagnostic kit for the detection of beta-lactamases or carbapenemases-type beta-lactamases as expressed by *Enterobacteriaceae* and non-Enterobacteriaceae, **characterized in that** it comprises an absorbent support impregnated with 1 to 1000µg of NXL104, alone or with 0.25-256 µg of a beta-lactam or carbapem antibiotic.

18. Diagnostic kit according to claim 17, **characterized in that** the support is a microtitre plate.

19. A method of treating an antibiotic resistant patient comprising administering NXL104 in combination with an antibiotic after detection of a beta-lactamase or carbapenemase-type beta-lactamase.

20. The method of claim 19, wherein the antibiotic is chosen from the group consisting of the penams, the penems, the carbapenems, the cephems, the cephalosporins, the carbacephems, the oxacephems, the cephamycins and the monobactams.

21. The method of claim 19, wherein the antibiotic is chosen from the group consisting of amoxicillin, ampicillin, azlocillin, mezlocillin, apalcillin, hetacillin, bacampicillin, carbenicillin, sulbenicillin, ticarcillin, piperacillin, azlocillin, mecillinam, pivmecillinam, methicillin, ciclacillin, talampicillin, aspoxicillin, oxacillin, cloxacillin, dicloxacillin, flucloxacillin, nafcillin, pivampicillin, cephalothin, cephaloridine, cefaclor, cefadroxil, cefamandole, cefazolin, cephalexin, cephradine, ceftaroline, ceftizoxime, cefoxitin, cephacetrile, cefotiam, cefotaxime, cefsulodin, cefoperazone, ceftizoxime, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidin, cefixime, ceftibuten, cefdinir, cefpodoxime axetil, cefpodoxime proxetil, cefteram pivoxil, cefetamet pivoxil, cefcapene pivoxil cefditoren pivoxil, cefuroxime, cefuroxime axetil, loracarbacef, latamoxef, imipenem, meropenem, biapenem, panipenem, aztreonam, carumonam and pharmaceutically acceptable salts thereof.

22. A method of treating a patient infected with a bacterium expressing a beta-lactamase or carbapenemase-type beta-lactamase comprising administering NXL104 in combination with an antibiotic.

23. The method of claim 22, wherein the antibiotic is chosen from the group consisting of the penams, the penems, the carbapenems, the cephems, the cephalosporins, the carbacephems, the oxacephems, the cephamycins and the monobactams.

24. The method of claim 22, wherein the antibiotic is chosen from the group consisting of amoxicillin, ampicillin, azlocillin, mezlocillin, apalcillin, hetacillin, bacampicillin, carbenicillin, sulbenicillin, ticarcillin, piperacillin, azlocillin, mecillinam, pivmecillinam, methicillin, ciclacillin, talampicillin, aspoxicillin, oxacillin, cloxacillin, dicloxacillin, flucloxacillin, nafcillin, pivampicillin, cephalothin, cephaloridine, cefaclor, cefadroxil, cefamandole, cefazolin, cephalexin, cephradine, ceftaroline, ceftizoxime, cefoxitin, cephacetrile, cefotiam, cefotaxime, cefsulodin, cefoperazone, ceftizoxime, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidin, cefixime, ceftibuten, cefdinir, cefpodoxime axetil, cefpodoxime proxetil, cefteram pivoxil, cefetamet pivoxil, cefcapene pivoxil cefditoren pivoxil, cefuroxime, cefuroxime axetil, loracarbacef, latamoxef, imipenem, meropenem, biapenem, panipenem, aztreonam, carumonam and pharmaceutically acceptable salts thereof.
